# EUROPÄISCHE PATENTSCHRIFT

(11) **EP 0 619 095 B1**
(45) Veröffentlichungstag und Bekanntmachung des Hinweises auf die Patenterteilung: **13.01.1999**
(21) Anmeldenummer: 94104476.0
(22) Anmeldetag: 22.03.1994
(51) Int. Cl.: A61B 5/117

(54) **Vorrichtung zur Erfassung von Oberflächenstrukturen**
Device for measurement of surface structures
Dispositif de mesure de structures superficielles

(30) Priorität: 30.03.1993 DE 4310390
(43) Veröffentlichungstag der Anmeldung: 12.10.1994
(73) Patentinhaber: Sonident Anstalt Liechtensteinischen Rechts, 9490 Vaduz (LI)
(72) Erfinder: Bicz, Wieslaw, PL-52-011 Wroclaw (PL)
(74) Vertreter: Funck-Hartherz, Anna-Eleonore, Dipl.-Phys.

(56) Entgegenhaltungen:
- EP-A- 0 048 489
- EP-A- 0 451 565
- DE-A- 3 610 397

## Beschreibung

Die Erfindung bezieht sich auf eine Vorrichtung zur Erfassung von Oberflächenstrukturen und oberflächennahen Strukturen unter Verwendung von Ultraschall, die sich insbesondere zur Ermittlung der Strukturen der Fingerkuppen eignet.

Vorrichtungen dieser Art unter Verwendung von Ultraschall sind bereits bekannt geworden. Gemäß der in der EP-A-0 402 779 beschriebenen Vorrichtung wird das auf einer ebenen oder gewölbten ultraschalldurchlässigen Auflage befindliche Objekt, z.B. eine Fingerkuppe, durch einen mit Flüssigkeit gefüllten Körper oder einen Festkörper mit Ultraschallwellen beschallt und die am Objekt zurückgestreuten und reflektierten Wellen werden von einem Empfänger aufgenommen. Durch die Intensität der rückgestreuten Wellen kann die Beschaffenheit der Oberfläche und der oberflächennahen Strukturen ermittelt werden. Der Sender der Ultraschallwellen und der Empfänger sind scheibenförmig und getrennt voneinander angeordnet, und es werden nur Sender gewählt, die ebene oder sphärische parallel zur Oberfläche verlaufende Wellen aufweisen und der Empfänger ist nur für solche empfindlich. Auf diese Weise erhält der Empfänger direkt eine Fouriertransformation, aus der jeder Parameter, wie Phase, Amplitude oder Intensität entnommen werden kann. Je nach der Struktur des zu untersuchenden Objektes variiert die Streuung und damit die Intensität der rückgestreuten Wellen in den verschiedenen Richtungen unterschiedlich stark. Den einzelnen Intensitätswerten können Meßeinheiten, wie Zahlen, Farben und dergleichen zugeordnet werden. Durch eine solche Zuordnung werden dann entsprechende Bilder der Strukturen des Objektes erhalten.

Es hat sich herausgestellt, daß mit den bekannten Vorrichtungen keine ausreichend guten und differenzierbaren Ergebnisse erzielt werden können, da diese bekannten Methoden zu stark von der Lage des jeweiligen Objektes abhängig sind. Die Erfindung hat sich deshalb die Aufgabe gesetzt, eine Ultraschallabbildungsvorrichtung zur Erfassung und/oder Identifikation von Oberflächenstrukturen und oberflächennahen Strukturen eines Objektes zu schaffen, die eine möglichst gleichmäßige Beschallung des Objektes und eine möglichst gute Wiedergabe der Oberflächenstrukturen und oberflächennahen Strukturen unabhängig von der Lage des Objektes ermöglicht.

Ausgehend von der vorher beschriebenen Vorrichtung wird dies gemäß der Erfindung mit einer Vorrichtung gemäß Anspruch 1 erreicht. Das dabei vorgesehene Loch (Pinhole) für die Strahlungsquelle hat einen geringen Durchmesser und liegt am Brennpunkt eines fokussierenden Sendewandlers für die Ultraschallwellen und ermöglicht eine sehr intensive und gleichmäßige Beschallung des gesamten Objektes. Der Durchmesser des Loches liegt mindestens in der Größenordnung der Ultraschallwellen und reicht bis zum 10-fachen derselben. Das Loch der Strahlungsquelle befindet sich in einem Träger und ist dabei soweit von der Auflagefläche des Objektes entfernt, daß dieses vollständig bestrahlt wird. Die vollständige Bestrahlung des Objektes ist eine Voraussetzung der erfindungsgemäßen Vorrichtung. Die lochförmige Begrenzung bedingt, daß nur sphärische Wellen auf das Objekt auftreffen. Die vom Sendewandler normalerweise ausgehenden Wellen sind nicht homogen, also nicht exakt sphärisch, jedoch gehört zur Funktionstüchtigkeit der Vorrichtung eine spektrale Reinheit und es sollen nur sphärische Wellen das Objekt erreichen.

In Weiterbildung der Erfindung wird vorgeschlagen, einen vorzugsweise ringförmigen Empfangswandler zu verwenden, der aus einer Vielzahl von kleinen punktförmigen Wandlern zusammengesetzt ist. Als Mindestzahl der Empfangswandler werden vorzugsweise 256 verwendet, die zu einem Ring zusammengesetzt sind.Es hat sich herausgestellt, daß die Durchmesser der aneinandergereihten Wandlerelemente in der Größenordnung der Wellenlänge des Ultraschalls liegen. Damit genügend Energie auf den Empfangswandlerring zurückgebeugt wird, wird vorgeschlagen, die Auflage für das Objekt als konkav-konvexe oder konvex-konkave Linse auszubilden, wobei das Objekt auf der konvex gewölbten Seite aufliegt. Anstelle einer Linse als Auflagefläche kann auch eine konvex-konkave Scheibe gleicher Dicke verwendet werden. Auf diese Weise wird eine fokussierende Wirkung in Richtung auf die Empfangswandler bzw. den Empfangswandlerring erreicht. Die Wellen, die nicht von dem Objekt zurückgebeugt werden, reflektieren zum Sender zurück.

Zur Ermittlung der Oberflächenstruktur und der oberflächennahen Strukturen wird das Objekt mit einer Impulsgruppe (Burst) bestrahlt. Es hat sich gezeigt, daß 3000 Impulsgruppen pro Sekunde gesendet werden können und zwar bei einem Abstand von ca. 10 cm zwischen dem Loch und der Auflage für das Objekt. Um das komplette Diffraktionsbild zu erhalten, ist es notwendig, nacheinander mit verschiedenen Frequenzen im Ultraschallbereich zu senden. Bei jeder Frequenz wird jeder Wandler einzeln nacheinander abgefragt. Die Abfragung erfolgt zweckmäßigerweise bei 20 verschiedenen Frequenzen im Ultraschallbereich. Diese Maßnahme ist notwendig, um die erforderliche Auflösung des Diffraktionsbildes zu erhalten.

Der weitere Aufbau der erfindungsgemäßen Vorrichtung ist derart, daß ein Frequenzgenerator eine Impulsgruppe mit vorgegebener Frequenz über einen Verstärker dem Sendewandler zuführt. Die von dem Objekt zurückgestreuten Wellen werden von dem Empfangswandler aufgenommen und als Signale an einen Verstärker weitergegeben. Die verstärkten Signale werden einem Detektor zugeführt, der die Signale in einen Gleichstrom umwandelt. Davon wird die durchschnittliche Amplitude in einem vorgegebenen Zeitabschnitt gemessen und einem Computer zur Auswertung zugeführt, der auch wegen der zeitlichen Koordination den Frequenzgenerator steuert.

Weitere Einzelheiten werden anhand der Zeichnungen erläutert. Dabei zeigen:
- Fig. 1: den schematischen Aufbau der erfindungsgemäßen Vorrichtung,
- Fig. 2: eine Draufsicht auf die Trägerfläche mit dem Empfangswandlerring,
- Fig. 3: eine vergößerte Darstellung des Bereiches A der Figur 2,
- Fig. 4: eine vergrößerte Darstellung des Bereiches B der Figur 1.

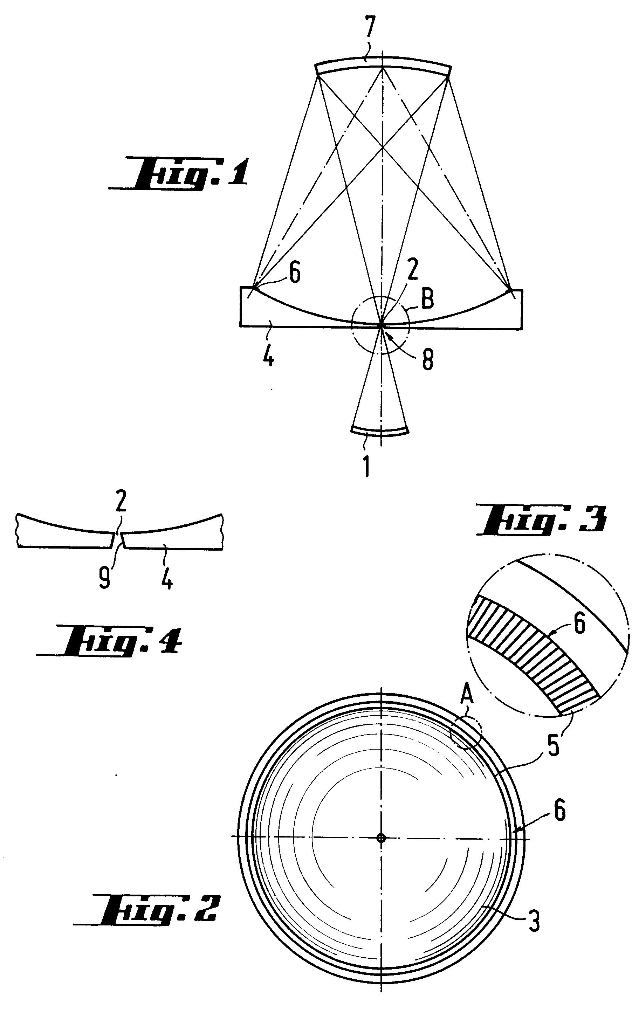

Fig. 1 zeigt den Aufbau der Vorrichtung vom Prinzip her. Der fokussierende Sendewandler 1 sendet Ultraschallwellen aus, die sich im Brennpunkt treffen und die die punktförmige Strahlungsquelle 8 bilden. Diese erscheint in dem Loch 2 des Trägers 4. Es hat sich herausgestellt, daß das Loch 2 am zweckmäßigsten von einer trichterförmigen Öffnung 9, wie in Fig. 4 dargestellt, gebildet ist.

Auf der Fläche 3 des Trägers 4 befindet sich, wie in den Figuren 2 und 3 gezeigt, der aus zahlreichen kleinen Wandlern 5 gebildete Empfangswandlerring 6. Im Bereich des Wandlerringes 6 ist die Fläche 3 des Trägers 4 sphärisch ausgebildet, deren Mittelpunkt in der Mitte der Auflagefläche der Auflage 7 liegt. Die Ausbildung der Fläche 3 kann beliebig sein, solange nur der Bereich des Empfangswandlers sphärisch ausgebildet ist. Gegenüber dem Wandlerring ist die als konvex-konkave auf den Empfangswandler 5 hin gerichtete Scheibe ausgebildete Auflage 7 für das Objekt vorgesehen. Anstelle der Scheibe kann auch eine Linse eingesetzt werden. Die konvexe Seite dient als Auflagefläche für das Objekt. Diese Auflage fokussiert die zurückgestreuten Ultraschallwellen auf den Empfangswandlerring 6. Auf diese Weise wird ausreichend Energie dem Empfangswandler zugeführt. Zwischen der Auflage 7 und der Fläche 3 des Trägers 4 befindet sich ein Medium, nämlich eine Flüssigkeit, z.B. Wasser oder ein Festkörper, z.B. Glas oder Plexiglas.

Der Strahlenverlauf der Ultraschallwellen ist in Fig. 1 skizziert. Die vom Sendewandler ausgesandten Ultraschallwellen fokussieren in dem Loch 2 und verlaufen weiter bis zur Auflage 7 durch das zwischen beiden befindliche Medium. Auf der konvexen Fläche der Auflage befindet sich das jeweilige Objekt, wobei sich die erfindungsgemäße Vorrichtung besonders zur Erfassung und Identifikation von der Haut der Fingerkuppen eignet, und zwar sowohl der Oberfläche als auch der unter der Oberfläche liegenden Hautstruktur, die das Wachstum der Haut verursacht. Die Vorrichtung gemäß der Erfindung ist so konstruiert, daß die gesamte Fläche der Auflage 7 beschallt wird. Als Folge der fokussierenden Wirkung der Auflage wird ein erheblicher Teil der zurückgestreuten Wellen auf den Empfangswandlerring 6 fokussiert, so daß ein einwandfreies Diffraktionsbild vermittelt wird.

Es ist nicht zwingend, das Loch 2 mittig in der Fläche 3 des Trägers 4 anzuordnen, jedoch muß garantiert sein , daß die vom Objekt zurückgestreuten Wellen auf den ringförmigen Empfangswandler treffen.

## Patentansprüche

1. Ultraschallabbildungsvorrichtung zur Erfassung und/oder Identifikation von Oberflächenstrukturen und oberflächennahen Strukturen eines auf einer Auflage liegenden Objektes, das durch eine Flüssigkeit oder einen Festkörper mit den Ultraschallwellen eines Sendewandlers (1) beschallt wird, wobei die durch das Objekt zurückgestreuten Ultraschallwellen von einem Empfangswandler (5) erfaßt werden,
**dadurch gekennzeichnet,** daß
a) der Empfangswandler (5) auf der der Auflage zugewandten sphärischen, konkaven Fläche (3) eines Trägers (4) angeordnet ist, der ein Loch (2) aufweist, dessen Durchmesser in der Größenordnung der Wellenlänge der Ultraschallwellen bis zum zehnfachen derselben liegt,
b) der Sendewandler (1) die von ihm ausgesandten Ultraschallwellen so fokussiert, daß deren Brennpunkt im Loch (2) des Trägers (4) liegt, so daß das Loch (2) zur gleichzeitigen Beschallung des gesamten Objektes als Strahlungsquelle (8) für sphärische Ultraschallwellen wirkt, und
c) der Krümmungsmittelpunkt der sphärischen Fläche (3) in der Mitte der Auflagefläche der Auflage (7) liegt, die fokussierend wirkt.

2. Vorrichtung nach Anspruch 1,
**dadurch gekennzeichnet,**
daß der Empfangswandler (5) aus einem oder mehreren Wandlerelementen (5) besteht, die um das Loch (2) herum angeordnet sind.

3. Vorrichtung nach Anspruch 1,
**dadurch gekennzeichnet,**
daß die Auflage (7) eine konvex-konkave oder eine konkav-konvexe Linse ist.

4. Vorrichtung nach Anspruch 1,
**dadurch gekennzeichnet,**
daß die Auflage (7) als konvex-konkave Scheibe gleicher Dicke ausgebildet ist.

5. Vorrichtung nach Anspruch 1 und 2,
**dadurch gekennzeichnet,**
daß die Wandlerelemente (5) aneinandergereiht sind.

6. Vorrichtung nach Anspruch 4,
**dadurch gekennzeichnet,**
daß die Wandlerelemente (5) in einem Ring (6) angeordnet sind.

7. Vorrichtung nach den Ansprüchen 5 oder 6,
**dadurch gekennzeichnet,**
daß die Durchmesser der aneinandergereihten Wandlerelemente (5) in der Größenordnung der Wellenlänge des Ultraschalls liegen.

8. Vorrichtung nach Anspruch 1,
**dadurch gekennzeichnet,**
daß die Ultraschallbestrahlung mit einer Impulsgruppe erfolgt.

9. Vorrichtung nach Anspruch 1,
**dadurch gekennzeichnet,**
daß die Ultraschallbestrahlung mit unterschiedlichen Frequenzen im Ultraschallbereich erfolgt.

10. Vorrichtung nach einem oder mehreren der vorhergehenden Ansprüche,
**dadurch gekennzeichnet,**
daß das Loch (2) im Mittelpunkt der Fläche (3) des Trägers (4) vorgesehen ist.

11. Vorrichtung nach den Ansprüchen 1 oder 8,
**dadurch gekennzeichnet,**
daß das Loch (2) von einer trichterförmigen Öffnung (9) des Trägers (4) gebildet ist.

## Claims

1. Device for depiction of ultrasonics to record and/or identify structures on the surface and nearby of an object laying on a support and being exposed to ultrasonic waves of a transmitting transducer (1) passing through a liquid or a solid and the ultrasonic waves scattered back from the object are recorded by a receiving transducer (5)
**characterized in that**
a) the receiving transducer (5) is placed on the spherical concave surface (3) of a support dircted to the support for the object with a hole (2) being from one to ten times of the wavelength of the ultrasonic waves in diameter.
b) the transmitting transducer (1) is focussing the ultrasonic waves transmitted by himself in this way that their focal point is located in the hole of the support (4) so that the hole (2) works as source of radiation (8) for spherical ultrasonic waves to expose the whole object simultaneously to ultrasonic waves and
c) the curvecenter of the spherical surface (3) is located in the center of the focussing support (7) surface.

2. Device according patent claim 1,
**characterized in that**
the receiving transducer (5) consists of one or several transducer elements (5) located arround the hole.

3. Device according patent claim 1,
**characterized in that**
the support (7) is a convex concave or concave convex lens.

4. Device according patent claim 1,
**characterized in that**
the support (7) consists of a convex concave disc of equal thickness.

5. Device according patent claim 1 and 2,
**characterized in that**
the transducer elements (5) are strung together.

6. Device according patent claim 4,
**characterized in that**
the transducer elements (5) are placed in a ring (6).

7. Device according patent claim 5 or 6,
**characterized in that**
the transducer elements strung together (5) are in the order of magnitude of the wavelength of the ultrasonics in diameter.

8. Device according patent claim 1,
**characterized in that**
the ultrasonic radiation is executed by means of a group of impulses.

9. Device according patent claim 1,
**characterized in that**
the ultrasonic radiation is executed with variable frequencies of ultarsonics.

10. Device according one or several of the preceding patent claims,
**characterized in that**
the hole (2) is located in the center of the surface (3) of the support (4).

11. Device according patent claims 1 or 8,
**characterized in that**
the hole (2) consists in a funnel shaped opening (9) of the support (4).

## Revendications

1. Dispositif à prendre des images ultrasoniques pour saisir et/ou identifier des structures superficielles et proche de la surface d'un objet posé sur un support qui est sonorisé avec des ondes ultrasonores d'un transducteur émetteur (1) à travers d'un liquide ou d'un materiel solide ou saisissant avec un transducteur recepteur (5) les ondes ultrasonores répandu par l'objet, caracterisé par le fait
a) que le transducteur récepteur (5) est arrangé sur le surface sphérique et concave (3) tourné vers le support d'objet, d'un autre support (4) avec un trou (2) dont le diamètre correspond à la longeur des ondes ultrasonores jusqu'a dix fois autant,
b) que le transducteur émetteur (1) met les ondes ultrasonores emis dans le point fecal d'une telle manière que leurs point fecal se trouve dans le trou (2) du support (4) et que le trou (2) serve comme élément radiateur (8) pour ondes ultrasonores sphériques à la sonorisation simultanée de tout l'objet, et
c) que le centre de la courbe de la surface sphérique (3) se trouve au milieu de la surface d'appui du support (7) focalisant.

2. Dispositif après revendication 1,
caracterisé par le fait,
que le transducteur récepteur (5) consiste d'un ou plusieurs éléments transducteurs (5) qui sont groupés autour du trou (2).

3. Dispositif après revendication 1,
caracterisé par le fait,
que le support (7) est une lentille convex-concave ou concave-convex.

4. Dispositif après revendication 1,
caracterisé par le fait,
que le support (7) consiste d'une disque convex-concave d'épaisseur égale.

5. Dispositif après revendication 1 et 2,
caracterisé par le fait,
que les éléments transducteurs (5) sont adjointes.

6. Dispositif après revendication 4,
caracterisé par le fait,
que les éléments transducteurs (5) sont groupés dans un cercle (6).

7. Dispositif après revendication 5 ou 6,
caracterisé par le fait,
que le diamètre des éléments transducteurs adjointes correspond à la longueur des ondes ultrasonores environ.

8. Dispositif après revendication 1,
caracterisé par le fait,
que la sonorisation ultrasonique est effectuée avec une groupe d'impusation.

9. Dispositif après revendication 1,
caracterisé par le fait,
que la sonorisation ultrasonique est effectuée avec des frequences differentes ultrasonique.

10. Dispositif après une ou plusieurs des revendication precedentes,
caracterisé par le fait,
que le trou (2) est prevu au centre de la surface (3) du support (4).

11. Dispositif après revendication 1 ou 8,
caracterisé par le fait,
que le trou (2) consiste d'une ouverture de la forme d'un cornet (9)dans le support (4).
